# EUROPEAN PATENT APPLICATION

(11) **EP 3 366 147 A1**
(43) Date of publication of application: **29.08.2018**
(21) Application number: 17842350.5
(22) Date of filing: 21.11.2017
(51) Int. Cl.: A23L 33/10, A23L 33/105, A61K 36/28, A61K 36/288, A61K 36/282

(54) **COMPOSITION FOR PROMOTING PERIODONTAL TISSUE REGENERATION**

(30) Priority: 30.12.2016 KR KR
(71) Applicant: Famenity Co., Ltd., Gwacheon-si, Gyeonggi-do 13837 (KR)
(72) Inventor: LEE, Ji Won, Gwacheon-si Gyeonggi-do 13834 (KR); NOH, Yoo Hun, Seoul 06347 (KR)
(74) Representative: Zardi, Marco
(86) International application number: PCT/KR2017/013257
(87) International publication number: WO 2018/124478

(57) **Abstract**

The present invention relates to a composition for promoting periodontal regeneration. The composition for promoting periodontal regeneration according to the present invention includes any one dicotyledon extract belonging to the order Campanula punctata, selected from the group consisting of Ussuri thistle extract, Helianthus annuus extract, Dandelion extract, Chrysanthemum extract, Arctium lappa extract, Common cosmos extract, Ligularia stenocephala extract, Foremost mugwort extract, Cichorium intybus extract, and mixture thereof.

## Description

### [Technical Field]

The present invention relates to a composition for promoting periodontal regeneration. More particularly, the present invention relates to a composition for promoting periodontal regeneration, which is capable of being useful for treating periodontal disease and regenerating damaged periodontal tissue by utilizing a natural extract capable of promoting periodontal regeneration to promote the differentiation of cells constituting the periodontium.

### [Background Art]

The periodontium is composed of gingiva, cementum, periodontal ligament, and alveolar bone, and thus all of the constituent tissue are different in nature so that the treatment for regenerating a lesion destroyed by periodontal disease does not show promising results yet. Although periodontal disease enters the healing stage through the non-surgical or surgical removal of plaque, tartar, and inflammatory connective tissue, these traditional approaches to treatment result in incomplete healing for the lost periodontium due to the proliferation of epithelial tissue, rather than the regeneration of individual components of the periodontium.

Thus, various methods are being developed to regenerate damaged periodontium and to treat periodontal diseases. Particularly, there is active research on periodontal regeneration using bone graft materials. For recovery of the lost alveolar bone tissue, for example, the autologous bone graft is used to induce osteogenesis by filling the damaged site, bone of a person or animal from which immunogenicity is removed is used as an artificial bone substitute material, or commercially available hydroxyapatite is used. Although the autogenous bone is known to be the best one, the obtainable bone tissue is localized, and its use is limited due to post-operative sequelae for obtaining the same. Allogenic bone, heterogeneous bone, and synthetic bone substitute are estimated to have low efficacy due to their low bone induction capacity. The heterogeneous bone or synthetic bone used as the bone graft material has relied on imported products until now, but domestic products are also being commercially available with recently strengthened domestic technology. However, periodontal ligament is not well regenerated, and new cementum is not well formed, which are essential for the periodontal regeneration despite the method of regenerating periodontium using the bone graft material, and thus the alveolar bone regeneration is often reported rather than the periodontal regeneration. In addition, since the shape of the damaged bone defective part is not fixed, a graft material capable of completely filling the bone defective part has not been developed until now.

As another method of regenerating periodontium, there is a method of using a bio-absorbable material or a non-absorbable material as a shield membrane for inducing tissue regeneration. By introducing the shield into the tissue, it is a fundamental concept to isolate the damaged site and the upper epithelial tissue to create an environment in which a new alveolar bone and periodontal ligament tissue are generated, and thus periodontal regeneration can occur relatively smoothly. If the damaged site is blocked from the surrounding environment with the shield as described above, the gingival fibroblasts cannot penetrate, and cells having regenerative power for bone and periodontal ligament are not interfered so that new periodontium is regenerated. However, when biocompatible non-absorbable materials such as silicone rubber, polyurethane, polyester, and expended polytetrafluoroethylene are introduced into the tissue through surgical procedures, it is troublesome that the subsequent removal surgery should be performed. In addition, there is a problem that abscess, epithelial down growth, periodontium formation, inflammation, and the like may occur in the neoplastic tissue during the second surgery. Thus, shields have been actively developed using bio-compatible materials absorbed *in vivo* recently. For example, a collagen shield, a natural polymer, can be applied to induction tissue regeneration or as a dressing material for skin or mucous tissue. However, there is a disadvantage that the antigenicity as a protein cannot be eliminated entirely to cause inflammation and allergic reaction, and the disintegration rate *in vivo* is not constant.

As related prior arts, there is patent document 1 (KR 10-1260757 B1, multilayer absorbable barrier membranes for guiding bone tissue regeneration) and the like.

When a biomaterial itself such as a bone graft material or a shield as described above is used as a graft material, although it can act as any mediator with bone conduction capacity itself, it does not have bone inductive strength for initial bone formation, which is essential for the shortening of the treatment period, thereby having a limitation of bone formation after a considerable period of time after surgery.

As related prior arts, Patent Document 2 (KR 10-2015-0053371 A, bone graft material for regeneration of periodontium and preparation method thereof) and the like may be referred.

Recently, attention has been focused on the effect of bone regeneration of demineralized bone powder and bone morphogenic protein (BMP) obtained therefrom. The only inducer for osseous tissue regeneration currently commercially available is Emdogain, which is composed of substances of enamel matrix derivatives, particularly amelogenin, to induce natural tooth growth, thereby inhibiting the formation of epithelial tissue in the defective part and forming new alveolar bone and cementum. However, since the amelogenin is prepared by extracting from developing teethgerm of 6-month-old young pig embryo, it is likely to cause an immune reaction when used in humans, and its production is small, and its price is high.

In addition, various methods have been tested to exhibit regenerative ability, but in practice, they are not widely applied to patients in the field of medicine, and thus treatments which are recognized as a procedure with high predictability of a good prognosis shall be developed.

In order to develop a method of periodontal regeneration with such high predictability, it may be necessary to base a wide range of cellular and molecular biologic understanding about treatment on periodontium wound. In general, the wound healing process on bloody injury occurs through four steps: hemostasis, inflammation, cleavage, and regeneration, and the healing process associated with periodontal treatment is similar thereto. When tissue injury associated with the periodontal disease progression and bloody periodontal treatment occurs, the blood components leak from the hemorrhagic blood vessels and the wounds are filled with blood and platelet aggregation. The blood clot consists of a fibrin fiber matrix and a growth factor incorporated thereinto. Here, the fibrin fiber matrix acts as a natural provisional matrix for tissue regeneration. When several cytokines and growth factors are secreted so that the proper environment is established by normal inflammation, mother cells that regenerate the periodontium will differentiate, thereby regenerating periodontal ligament, alveolar bone, and cementum.

Therefore, it is necessary to develop a composition that can increase the self-proliferating activity of periodontal stem cells *in vivo* so as to promote the periodontal regeneration, which is based on the cellular and molecular biologic studies on the role of fibrin during wound healing of teeth and periodontium. Particularly, by promoting the periodontal regeneration, the inflammation of the periodontium damaged by periodontal disease is inhibited, and the osteoblast cells are differentiated to obtain them in a significant amount, thereby enhancing alveolar bone tissue so that it is possible to restore the damage caused by periodontal disease.

### [Disclosure]

### [Technical Problem]

An object of the present invention is to provide a composition for promoting periodontal regeneration. Thus, it is possible to efficiently regenerate a tissue damaged by periodontal diseases.

Further, the present invention uses the composition to promote the autogenous proliferation of periodontal stem cells present *in vivo* and to promote the differentiation of stem cells for restoring gum and periodontal damage, thereby inhibiting inflammation of periodontium and promoting osteoblast differentiation so as to allow the regeneration and reinforcement of the alveolar bone tissue. Therefore, the tissue damaged by the periodontal disease is regenerated efficiently to ultimately allow periodontium to be reconstructed.

In addition, the composition for promoting periodontal regeneration of the present invention promotes the proliferation of periodontal ligament stem cells not only in damaged periodontium but also in healthy periodontium so that the periodontium can be formed more densely, thereby promoting periodontal health.

### [Technical Solution]

In order to achieve the objects, a composition for promoting periodontal regeneration according to an embodiment of the present invention may include any one dicotyledon extract belonging to the order Campanula punctata, selected from the group consisting of Ussuri thistle extract, Helianthus annuus extract, Dandelion extract, Chrysanthemum extract, Arctium lappa extract, Common cosmos extract, Ligularia stenocephala extract, Foremost mugwort extract, Cichorium intybus extract, and mixture thereof.

Further, a composition for promoting periodontal regeneration may include Ligularia stenocephala extract and any one dicotyledon extract belonging to the order Campanula punctata, selected from the group consisting of Ussuri thistle extract, Helianthus annuus extract, Dandelion extract, Chrysanthemum extract, Arctium lappa extract, Common cosmos extract, Foremost mugwort extract, Cichorium intybus extract, and mixture thereof.

In the composition for promoting periodontal regeneration, the dicotyledon extract belonging to the order Campanula punctata may be extracted with any one solvent selected from the group consisting of water, an organic solvent having 1 to 10 carbon atoms, a subcritical fluid, a supercritical fluid, and a mixture thereof.

A food composition according to another embodiment of the present invention may include the composition for promoting periodontal regeneration.

A pharmaceutical composition according to another embodiment of the present invention may include the composition for promoting periodontal regeneration.

Hereinafter, the present invention will be described in detail.

The composition for promoting periodontal regeneration according to an embodiment of the present invention may include any one dicotyledon extract belonging to the order Campanula punctata, selected from the group consisting of Ussuri thistle extract, Helianthus annuus extract, Dandelion extract, Chrysanthemum extract, Arctium lappa extract, Common cosmos extract, Ligularia stenocephala extract, Foremost mugwort extract, Cichorium intybus extract, and mixture thereof.

Throughout this specification, when an element is referred to as "including," it means that it may include other elements as well, without excluding the other elements unless expressly described otherwise.

Further, a composition for promoting periodontal regeneration may include Ligularia stenocephala extract and any one dicotyledon extract belonging to the order Campanula punctata, selected from the group consisting of Ussuri thistle extract, Helianthus annuus extract, Dandelion extract, Chrysanthemum extract, Arctium lappa extract, Common cosmos extract, Foremost mugwort extract, Cichorium intybus extract, and mixture thereof.

The term "periodontium" described in the present invention includes four types of tissues surrounding teeth and refers to act as physically supporting teeth, detecting movement of teeth, and supplying blood flow and the like required for teeth, which is defined as meaning all or each of gingiva (gum), cementum, periodontal ligament, and alveolar bone.

Meanwhile, the periodontal disease described in the present invention is recognized as one including gingivitis in which the inflammation is limited to soft tissues and periodontitis in which the inflammation extends to the periphery of the gum bone and the gum and is defined as one including periodontal ligament diseases.

The term "periodontal ligament disease" described in the present invention is defined as an inflammatory disease accompanied with bone loss and periodontal ligament inflammation in which a periodontal pocket is formed deeply when the periodontal inflammation is worse.

In general, periodontal disease is an infectious disease caused by periodontal pathogens in the plaque and is generally accompanied by tissue destruction due to chronic inflammation. Therefore, aside from improving the periodontal disease by eliminating bacterium and so on that cause periodontal disease, regenerating the periodontium destructed or damaged due to the periodontal disease is vital to prevent the periodontal disease from deteriorating and thus make the disease to be treated ultimately. In particular, it is considerably crucial that the periodontium regeneration is promoted to allow periodontium to be more densely differentiated, thereby increasing the resistance of periodontium.

Ussuri thistle is also called thorn and grows in mountains and fields. The stems stand straight and have a height of 50 to 100 cm with white hairs and spider-like hairs entirely. Further, the root leaves remain until the flower buds and are larger than the stem leaves. The stem leaves have a needle-shaped elliptical shape to split like a feather. The lower part thereof is wrapped around the base, and the split edge is split again. There is a thorn in a sawtooth having a deeply caved shape. The flowers bloom from June to August, and the color thereof is purple to red. The flowers are hanging on the end of stems and branches. Involucral bracts of the involucre are arranged in 7 or 8 rows, and the inside thereof is longer. The hairs of stamen are 16 to 19 mm in length. Mild plants are used as herbs, and mature roots are used as medicine. The fruit is 3.5 to 4 mm as an achene. Meanwhile, Ussuri thistle is distributed in Korea, Japan, Northeastern China, and Ussuri. Ussuri thistle with the narrow leaf is referred that the leaves are narrow and green, and the thorns thereof are somewhat more numerous. Thorny Ussuri thistle is referred that the leaves are attached densely, and the thorns thereof are more numerous. White thorny Ussuri thistle is referred that white flowers bloom.

Helianthus annuus is called a sun-oriented flower, Sanjayeon, morning-sun flower, which grows well everywhere, but grows particularly well in sunny places. It is native to central America and is widely planted therein. It grows up to about 2 m in height and has harsh hairs. The leaves are alternate phyllotaxis, have a long petiole, heart-shaped ovate, with a sawtooth on edge thereof. The flowers bloom in August and September, and the main stem hangs in one per the end of the branch and extends to the side. The flowers are 8 to 60 cm in diameter. The Ungulate flower is yellow and neutral, and the tubular flower is brown or yellow and bisexual. The fruit ripens in October, with two ridgelines, an inverted egg-shape, about 1 cm in length, and a black stripe on a gray background. Seeds contain 20 to 30% oil and are edible. It is planted for ornamental purposes. The inside of the stem is used as a medicinal material for diuretic, antitussive, and hemostasis. The varieties include ornamental plants and cultivars. The cultivar is planted especially in Russia and is planted in central and eastern Europe, India, Peru, and northern China. The residue is used as feed.

Dandelion is a plant belonging to the class dicotyledon of the herbaceous plant phylum, which grows in a sunny place in the field. There is no stem, and the leaves grow to be gathered from the roots and spread to the side. The leaves have an inverted needle-typed shape, 6 to 15 cm in length, 1.2 to 5 cm in width, deeply pitted with a feather-shape, with a sawtooth on edge thereof, and a few hairs. The flowers bloom with a color of yellow in April and May, and the compound flower is on the end of a flower stalk whose length is similar to that of the leaf. The white hairs are in the flower stalk but gradually disappear, leaving only the hairs under the compound flower. The involucre is 17 to 20 mm in length when blossomed, and the outer involucre bract has a narrow egg-shape or wide needle-shape, stands straight, and has horn-shaped protrusions at the end thereof. Fruits are achene, a long elliptical shape with a length of 3 to 3.5 mm, brown, and have thorn-like projections on the upper part thereof. The roots are 7 to 8.5 mm in length, and the hairs of stamen are 6 mm in length and have a color of light white. In spring, young leaves are eaten as herbs.

Chrysanthemum includes standard chrysanthemum and spray chrysanthemum depending on the flowering type. Standard chrysanthemum is such that one flower blooms on a single peduncle, and white and yellow flowers are often distributed in Korea, which are often used for funerals and rituals. The spray chrysanthemum is such that several flowers bloom on one peduncle, are usually used for flower arrangements or bouquets, in which chrysanthemum having various colors seen in the market and flower garden has been cultivated. Chrysanthemum exhibits a wide range of colors and shapes including most commonly seen single-flower type as well as various types such as double-flower type, anemone type, pompon type such as table tennis ball, and spider type like thinly elongated spider-web. Recently, there are many chrysanthemums for pot plant (less than 30 cm in length) used for interior decoration. Further, it is a perennial plant that is very strong in cold weather and can be wintered in the open field. Thus, it has a characteristic that flower buds are differentiated when the daytime length is less than 12 hours.

Arctium lappa is 50 to 150 cm high. Straight roots thereof grow 30 to 60 cm, and the stems come out from the end thereof. The leaves on the root come out in a group, and the petiole is long. They have staggered arrangement with a heart shape in the stems. The surface thereof is dark green, but white soft hair is densely on the back thereof. There are serrated teeth on edge thereof. The flowers bloom in July and August, with blackish purple color. The compound flowers hang on the end of the branch with corymb. The involucre is round, the bract is needle-shaped, and the tip thereof is like a hook. The flowers are tubular, the seeds are black, and the hairs of stamen are brown. The fruits are achenes and ripen in September. It is strong to have little illness, is very strong in cold weather, and does not depend on the quality of the soil. Breeding is carried out by seeding or dividing heads thereof. It is a naturalized plant of European origin. The varieties include Nongyacheon having long and thick roots and Sacheon having fleshy short roots. For recipes, it is made to pickles or boiled and thus eaten as side dishes. The roots contain inulin and some palmitic acid. In Europe, it is used as a diuretic and perspirant. Seeds are used as a diuretic when there is a swelling, and as an antidote to a sore throat and poison. It grows much in Japan and is wild in Europe, Siberia, and northeastern China.

Common cosmos is native to Mexico and is often planted for ornamental purposes. The stems are 1 to 2 m in height. The branches are split at the upper part thereof. The stems have no hair. The leaves are opposite to each other and are split in a shape of quill at two times and the split pieces are in a shape of a line. The flowers bloom from June to October, and there is one compound flower at each end of branches and stems. The compound flower is 6 cm in diameter and consists of 6 to 8 lingulate flowers and a yellow tubular flower. The color of lingulate flowers is very diverse, including light pink, white, and red, and the ends of the petals are lightly divided in a sawtooth-shape. The anthers of the tubular flower are dark brown and bear fruits. Involucre pieces are arranged in 2 rows and are a needle-shape with egg-shape, and the end thereof is pointed. The fruits are achenes, have no hairs, and have a beak-shape at their ends.

Foremost mugwort is also called wormwood, Sajaebal mugwort, or Mogitae mugwort. It grows in a sunny grass and has a height of 60 to 120 cm. Among the plants belonging to the genus Artemisia, all plants which are similar to Foremost mugwort in appearance are called Foremost mugwort. Among them, the species used for moxibustion is distinguished as "Artemisia dubia Wall." Foremost mugwort is hardly distinguishable because it is almost similar, but it is divided by the size of a head flower and the shape of a leaf. Artemisia dubia Wall is similar to Foremost mugwort, but distinguishes by its white hairy point on the leaf surface. Foremost mugwort refers to the most common type among Foremost mugwort-likes. There is a ridge on the stem, and the entire surface is covered with spider-like hairs. The root stalks extend sideways and shoots come out in a group. The leaves on the stem are staggered, have fake-stipule leaves, have an elliptical shape, and have 6 to 12 cm in length and 4 to 8 cm in width. It is split like feathers, with 2 to 4 pairs of cut pieces, but as it goes up, the leaves become smaller, and the number of cut pieces becomes smaller so that it becomes pure leaves. The leaves on the flower ear are in the shape of a line.

The flowers bloom in July to September with light ruddy purple, 2.5 to 3.5 mm in length. The head flower is one-sided, and the whole thereof is conical. The involucre is a long elliptical bell-shape, 2.5 mm in length, 1.5 mm in diameter, and has spider-like hairs. The bracts are lined with 4 lines. An outer bract is egg-shaped, and inner bract is long oval-shaped. The fruits are achenes and ripened in October, and their length are about 1.5 mm. Breeding is done by seeding, breaking, or dividing the heads thereof.

Cichorium intybus is native to northern Europe. The roots are fleshy and long, the stems are 50 to 150 cm in height and hard, and the branches are split and hairy. The leaves from roots face downward and split like a feather-shape. The split pieces are gradually narrowed at the bottom, have petioles like wings, and have large pieces at the end and triangular pieces at the side. The leaves on the stem have a needle-typed egg-shape or needle-shape, with a flat edge and hairs on the back thereof. The flowers bloom in July to September in light blue color, and lingulate flowers hang in the axillary stem of the upper part of the stem and the tip of the stem capitularly. The involucre is cylindrical, and the involucre bracts are divided into two pieces. Some varieties have white or light red flowers. Fruits are achenes, have a long ax-shape, and have 3 to 5 corners on the top thereof. The hairs of stamen are short, have a scale-shape, and have a slightly split end. It is cultivated much because it grows strong and adapts well to the environment. The roots are cooked a little bit and are eaten with butter, and the leaves are eaten as a salad. The young leaves that grow in the roots are collected in spring. Plants are used as feed or grass. The flowers are used as stimulants in the central nervous system and as drugs to enhance cardiac activity.

Ligularia stenocephala is a perennial herbaceous plant of dicotyledonous plant in the order Campanula punctata and the family Chrysanthemum, grows in the wetlands of deep mountains, and is 60 to 100 cm in height. There are no hairs in the whole grass of Ligularia stenocephala. The root stalks are thick. The leaves on the roots remain until the flowers bloom and are about 24 cm in length and 20 cm in width. The hairs grow along the back vein of the leaf and have pointed serrations on edge thereof. The petioles have 40 cm in length with no wings and broad base. The flowers are yellow, hanging from the tip of the stem racemosely in August to September. The fruits are achenes, ripen in October, and have an inverted needle-shape. Its color is brownish white with 6 to 7 mm long. Ligularia stenocephala is distributed in Korea, Japan, Taiwan, China, and the like. The young leaves are used for food, and the roots are used for treating women's diseases.

Stimulation on periodontal ligament cells and regeneration of whole tissues including periodontal ligament through the same are the most important in treatments for tissue reconstruction for periodontal disease, which causes damage of connective tissue and direct and indirect damage of tooth supporting tissues. However, periodontal ligament cells generally damaged first by periodontal disease are provided from periodontal ligament stem cells, which are adult stem cells.

Thus, the activation of adult stem cells of periodontium is recently the most critical target of reconstructive medicine for such periodontium reconstruction.

The composition for promoting periodontal regeneration according to the present invention may promote the effect that stem cells derived from periodontium generally maintain the tissue and regenerate the damage as an adult stem cell, thereby reconstructing periodontium.

Preferably, the composition for promoting periodontal regeneration may be a Ligularia stenocephala extract. In the case of using the Ligularia stenocephala extract, the effect of activating autogenous proliferation of periodontium and osteoblast stem cells is remarkably excellent compared to extracts of dicotyledon belonging to the order Campanula punctata. The effect of promoting periodontium regeneration, damaged due to periodontal disease is excellent. In addition, the Ligularia stenocephala extract has the more excellent effect of inhibiting inflammation of periodontium and promoting osteoblast differentiation to strengthen and regenerate periodontal bone tissue, thereby reconstructing periodontium compared to extracts of dicotyledon belonging to the order Campanula punctata.

Further, the composition for promoting periodontal regeneration is excellent in the transference of stem cells and ability to transfer to damaged tissue, so that regeneration effect on the real tissue can be obtained.

Therefore, when the Ligularia stenocephala extract is used, the regenerating activity on periodontium can be significantly promoted so that the composition for promoting periodontium regeneration has an excellent effect as compared with other extracts.

In the composition for promoting periodontal regeneration, the dicotyledon extract belonging to the order Campanula punctata may be extracted with any one solvent selected from the group consisting of water, an organic solvent having 1 to 10 carbon atoms, a subcritical fluid, a supercritical fluid, and a mixture thereof.

The Ligularia stenocephala extract may be extracted with any one solvent selected from the group consisting of water, an organic solvent having 1 to 10 carbon atoms, a subcritical fluid, a supercritical fluid, and a mixture thereof.

Preferably, the Ligularia stenocephala extract and the dicotyledon extract belonging to the order Campanula punctata may be extracted using a solvent in which water and ethanol are mixed. In the case of extracting the Ligularia stenocephala extract using the solvent, there is the advantage that the effect of inhibiting the inflammation of periodontium is enhanced as well as increasing the effect of enhancing activity of autogenous proliferation of periodontal stem cells and the effect of promoting the differentiation of stem cells to recover gum and periodontal damage.

The Ligularia stenocephala extract and the dicotyledon extract belonging to the order Campanula punctata may be one spray-dried or lyophilized after extract and may be provided in a uniform formulation that can be used by those skilled in the art, including a method of mixing dextrin. Further, the Ligularia stenocephala extract and the dicotyledon extract belonging to the order Campanula punctata may be one spray-dried or lyophilized after extract and may be provided in a uniform formulation that can be used by those skilled in the art, including a method of mixing dextrin.

A food composition according to another embodiment of the present invention may include the composition for promoting periodontal regeneration.

A pharmaceutical composition according to another embodiment of the present invention may include the composition for promoting periodontal regeneration.

### [Advantageous Effects]

A composition for promoting periodontal regeneration according to the present invention enables effective regeneration of tissues damaged due to periodontal disease.

Further, the composition for promoting periodontal regeneration according to the present invention activates autogenous proliferation of periodontal stem cells present *in vivo,* promotes differentiation of stem cells to recover gum and periodontal damage, thereby inhibiting inflammation on periodontium and promotes differentiation of osteoblast, thereby regenerating alveolar bone tissue while being capable of strengthening the same. Therefore, there is an effect that the tissue damage due to periodontal disease can be regenerated efficiently, and the periodontium can be restored ultimately.

In addition, the composition for promoting periodontal regeneration according to the present invention has the advantage of promoting the proliferation of periodontal ligament stem cells not only in damaged periodontium but also in healthy periodontium so that the periodontium is formed more densely, thereby enhancing periodontal health.

### [Description of Drawings]

FIG. 1 illustrates the anti-inflammatory effect of a composition for promoting periodontal regeneration according to an embodiment of the present invention.
FIG. 2 illustrates a test result of stem cell proliferation effect of a composition for promoting periodontal regeneration according to an embodiment of the present invention.
FIG. 3 illustrates mRNA expression effect of a composition for promoting periodontal regeneration according to an embodiment of the present invention.
FIG. 4 illustrates mRNA expression effect of the composition for promoting periodontal regeneration according to an embodiment of the present invention.
FIG. 5 illustrates mRNA expression effect of a composition for promoting periodontal regeneration according to an embodiment of the present invention.
FIG. 6 illustrates mRNA expression effect of a composition for promoting periodontal regeneration according to an embodiment of the present invention.
FIG. 7 illustrates a test for promotive effect on the damage-recovery ability of periodontal stem cells according to an embodiment of the present invention.

### [Best Mode]

The extract obtained by repeatedly extracting 2 to 8 times at an extraction temperature of 100°C for 2 to 8 hours was filtered with a filter cloth and concentrated in vacuum. Then the extract and dextrin were mixed and spray-dried to prepare Ussuri thistle extract.

Meanwhile, Ussuri thistle extract, Helianthus annuus extract, Dandelion extract, Chrysanthemum extract, Arctium lappa extract, Common cosmos extract, Ligularia stenocephala extract, Foremost mugwort extract, and Cichorium intybus extract were spray-dried under the same conditions and methods as those of Ussuri thistle extract. For other test conditions, the mixture of water and ethanol was used as a solvent.

### [Experimental Example 1: Test for anti-inflammatory effect]

For purpose of confirming the anti-inflammatory effect of each extract in addition to the activity for periodontium reconstruction, in order to find effect on change of Interleukin-1 (IL-1) and tumor necrosis factor (TNF-α) levels associated with the inflammation of gingivitis and the destruction of connective tissue, human-derived THP-1 was cultured in a culture medium supplemented with 10% FBS in RPMI 1640, and then the cell strains were co-treated with lipopolysaccharide (LPS), an inflammation-inducing substance, and each extract according to the present invention. Then, the secreted amount of TNF-α in the culture solution was measured using enzyme-linked immunosorbent assay (ELISA).

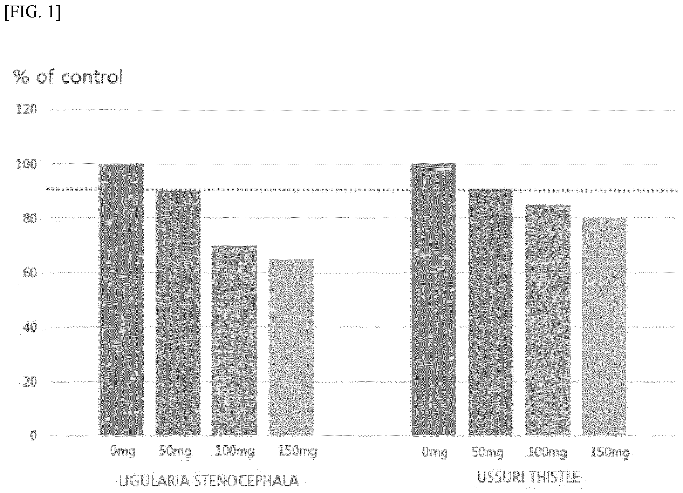

Referring to FIG. 1, as a result thereof, it was confirmed that each extract had a specific anti-inflammatory effect. However, as a result of conducting experiments at a concentration range of 50 to 150 mg/ml, it was found that Helianthus annuus extract, Dandelion extract, Chrysanthemum extract, Arctium lappa extract, Common cosmos extract, Foremost mugwort extract, and Cichorium intybus extract did not exhibit an anti-inflammatory effect of more than 90%. On the other hand, in the case of Ussuri thistle extract and Ligularia stenocephala extract, it was confirmed that the anti-inflammatory effect was increased depending on the concentration range of each extract. In particular, in the case of the group in which 50 mg/ml of the Ligularia stenocephala extract was also put exhibited anti-inflammatory effect with more than 90%. Thus, it was confirmed that the Ligularia stenocephala extract had the best anti-inflammatory effect in each concentration range.

Therefore, it can be found that the use of the Ligularia stenocephala extract reduces the amount of TNF-α secreted, and thus the Ligularia stenocephala extract exhibits a remarkably excellent anti-inflammatory effect in the periodontal disease.

### [Modes of the Invention]

### [Experimental Example 2: Test for proliferation effect of periodontal ligament stem cells by Ligularia stenocephala extract]

Stem cells derived from periodontium are adult stem cells, which generally act as maintaining the tissue *in vivo* and regenerating the damage. Therefore, in order to confirm the effect of Ligularia stenocephala extract on the periodontal ligament stem cells in periodontal reconstruction using the Ligularia stenocephala extract having the best anti-inflammatory effect, the test was conducted for the effect of the Ligularia stenocephala extract on the promotion of proliferation of periodontal ligament stem cells.

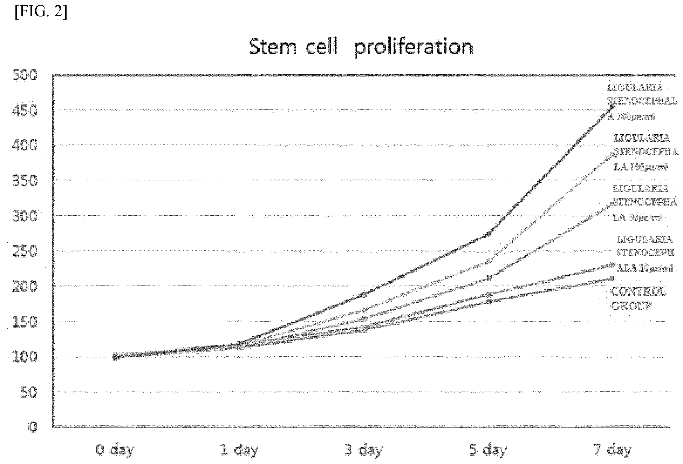

In order to confirm the effect on the reconstruction of oral cavity tissue by healthy self-division of periodontal ligament stem cells, human-derived periodontal stem cells were cultured with a culture medium in which Dulbecco's modified eagle medium (DMEM) was supplemented with 10% heat-inactivated fetal bovine serum. Then, the first cultured stem cells were treated with or without the Ligularia stenocephala extract of the present invention, and then the cell proliferation was confirmed by MTT assay on the periodontal cells. The results are illustrated in FIG. 2.

Referring to FIG. 2 as described below, it can be seen that the proliferation of periodontal ligament stem cells was improved depending on the concentration thereof when the treatment with the Ligularia stenocephala extract according to the present invention. Therefore, it can be seen that the Ligularia stenocephala extract improves proliferation of periodontal ligament stem cells, thereby promoting periodontal regeneration.

### [Experimental Example 3: Measurement of expression of MYC, KLF4, NOTCH1, and PCNA gene in the Ligularia stenocephala extract]

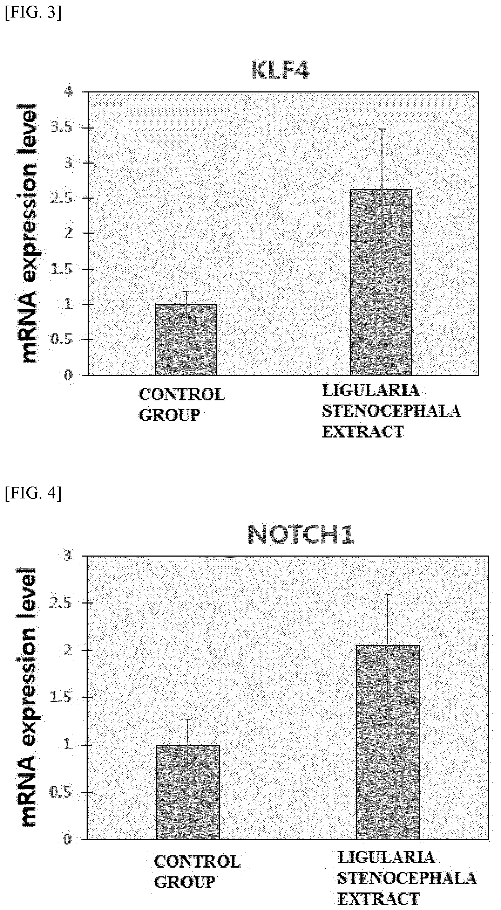

MYC, KLF4, NOTCH1, and PCNA genes have very close effects on the proliferation of periodontal ligament stem cells. Therefore, in order to confirm a change in the expression of the above-described genes by the Ligularia stenocephala extract according to the present invention, the expression of the gene mRNA was confirmed and then the results thereof are illustrated in FIGS. 3 to 6 as described below.

Referring to FIGS. 3 to 6 as described below, it can be confirmed that the expression of the above-described genes is promoted in the case of using the Ligularia stenocephala extract. In particular, referring to FIGS. 3 and 4, the expression of KLF4 and NOTCH1, which are biomarkers of periodontal ligament stem cells, is 2.6 times and 2.1 times, respectively, higher than that of the control group when treated with a concentration of 200 µg/ml of Ligularia stenocephala extract. This is the result showing the proliferation of the stem cells when the state of the stem cell is maintained.

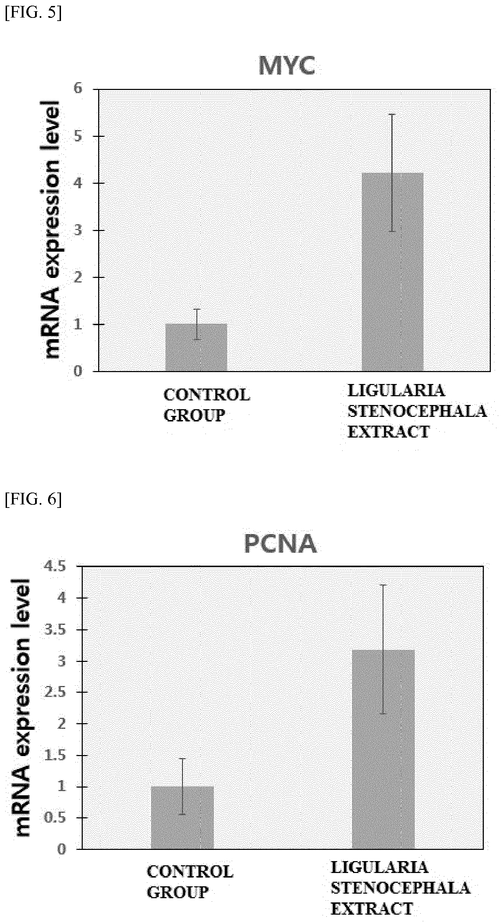

Further, referring to FIGS. 5 and 6, the expression of MYC and PCNA, genes involved in the proliferation of periodontal ligament stem cells was increased in 4.2 times and 3.2 times higher than that of the control group, respectively, when treated with a concentration of 200 µg/ml of the Ligularia stenocephala extract. As a result, it is shown that Ligularia stenocephala may enhance the proliferation of stem cells of periodontal ligament through MYC and PCNA genes, which are generally increased when stem cells proliferate.

### [Experimental Example 4: Test for effect of promoting damage recovery ability of periodontal stem cells by Ligularia stenocephala extract]

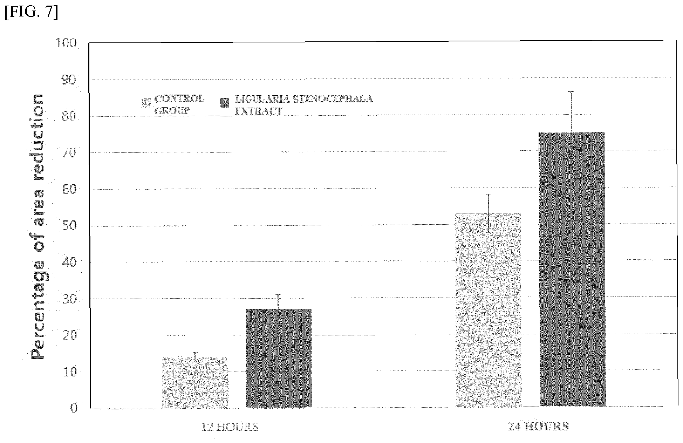

In order to confirm whether the Ligularia stenocephala extract according to the present invention can provide a regenerating effect of the real tissue, the scratch damage analysis model was used to conduct a test for the transference of stem cells and ability to transfer to damaged tissue for the Ligularia stenocephala extract. The results are illustrated in FIG. 7 as described below.

Referring FIG. 7 as described below, the ability to transfer of the periodontal ligament stem cells treated with the Ligularia stenocephala extract having a concentration of 200 µg/ml and the periodontal ligament stem cells as a control group was analyzed. As a result, it was confirmed that the periodontal ligament stem cells treated with the Ligularia stenocephala extract were more quickly transferred to the damaged area, thereby reducing the scratch area.

Further, in the case of the periodontal ligament stem cells treated with the Ligularia stenocephala extract, the damage recovery ability was such that the damaged area was recovered by about 27.34% for 12 hours, while the control group had the damage recovery ability of about 15.33% for 12 hours. Further, it was confirmed that the periodontal ligament stem cells treated with the Ligularia stenocephala extract showed a recovery of 75.32% after 24 hours, whereas the cells of the control group were recovered only 52.12% after 24 hours. In other words, this result shows that the Ligularia stenocephala extract can increase the regeneration effect of damaged tissue through the enhancement of the ability to transfer of stem cells.

While the preferred embodiments of present invention have been described in detail as described above, the present invention is not limited to the embodiments, and various modifications and improvements using the basic concept of the present invention, defined in the following claims by those skilled in the art are also within the scope of the present invention.

## Claims

1. A composition for promoting periodontal regeneration, the composition comprising any one dicotyledon extract belonging to the order Campanula punctata, selected from the group consisting of Ussuri thistle extract, Helianthus annuus extract, Dandelion extract, Chrysanthemum extract, Arctium lappa extract, Common cosmos extract, Ligularia stenocephala extract, Foremost mugwort extract, Cichorium intybus extract, and mixture thereof.

2. A composition for promoting periodontal regeneration, the composition comprising Ligularia stenocephala extract and any one dicotyledon extract belonging to the order Campanula punctata, selected from the group consisting of Ussuri thistle extract, Helianthus annuus extract, Dandelion extract, Chrysanthemum extract, Arctium lappa extract, Common cosmos extract, Foremost mugwort extract, Cichorium intybus extract, and mixture thereof.

3. The composition of claim 1, wherein the dicotyledon extract belonging to the order Campanula punctata is extracted with any one solvent selected from the group consisting of water, an organic solvent having 1 to 10 carbon atoms, a subcritical fluid, a supercritical fluid, and a mixture thereof.

4. The composition of claim 2, wherein the dicotyledon extract belonging to the order Campanula punctata is extracted with any one solvent selected from the group consisting of water, an organic solvent having 1 to 10 carbon atoms, a subcritical fluid, a supercritical fluid, and a mixture thereof.

5. A food composition including the composition for promoting periodontal regeneration according to claim 1.

6. A pharmaceutical composition including the composition for promoting periodontal regeneration according to claim 1.
